# EUROPEAN PATENT APPLICATION

(11) **EP 2 238 826 A1**
(43) Date of publication of application: **13.10.2010**
(21) Application number: 08857805.9
(22) Date of filing: 04.12.2008
(51) Int. Cl.: A01H 5/00, A01G 7/00, A01H 1/00, C12N 15/09

(54) **TRANSGENIC PLANT CAPABLE OF FLOWERING AT LOW LIGHT INTENSITY**

(30) Priority: 07.12.2007 JP 2007317643
(71) Applicant: Suntory Holdings Limited, Kita-ku, Osaka-shi Osaka 530-8203 (JP)
(72) Inventor: TANAKA, Yoshikazu, Mishima-gun Osaka 618-8503 (JP)
(74) Representative: Vossius & Partner
(86) International application number: PCT/JP2008/072015
(87) International publication number: WO 2009/072542

(57) **Abstract**

The invention provides a method for producing a flowering plant which can flower at a low light intensity. Specifically, the invention relates to a transgenic plant which can flower in a low light environment; a method for producing the same; a method for inducing the same to flower; and so on. More specifically, the invention relates to a transgenic plant which is engineered so as to flower in a low light environment (e.g., indoors) by introducing a FT gene thereinto; a method for producing the same; a method for inducing the same to flower; and so on.

SEQUENCE LISTING

<110>  SUNTORY HOLDINGS LIMITED

<120>  Transgenic plant capable of flowering under low light intensity

<130>  S1991 EP S3

<140>  EP 08 85 7805.9
<141>  2008-12-04

<150>  JP 2007-317643
<151>  2007-12-07

<160>  4

<170>  PatentIn version 3.4

<210>  1
<211>  525
<212>  DNA
<213>  Arabidopsis thaliana


<220>
<221>  CDS
<222>  (1)..(525)

<400>  1
atg tct ata aat ata aga gac cct ctt ata gta agc aga gtt gtt gga       48
Met Ser Ile Asn Ile Arg Asp Pro Leu Ile Val Ser Arg Val Val Gly
1               5                   10                  15

gac gtt ctt gat ccg ttt aat aga tca atc act cta aag gtt act tat       96
Asp Val Leu Asp Pro Phe Asn Arg Ser Ile Thr Leu Lys Val Thr Tyr
            20                  25                  30

ggc caa aga gag gtg act aat ggc ttg gat cta agg cct tct cag gtt      144
Gly Gln Arg Glu Val Thr Asn Gly Leu Asp Leu Arg Pro Ser Gln Val
        35                  40                  45

caa aac aag cca aga gtt gag att ggt gga gaa gac ctc agg aac ttc      192
Gln Asn Lys Pro Arg Val Glu Ile Gly Gly Glu Asp Leu Arg Asn Phe
    50                  55                  60

tat act ttg gtt atg gtg gat cca gat gtt cca agt cct agc aac cct      240
Tyr Thr Leu Val Met Val Asp Pro Asp Val Pro Ser Pro Ser Asn Pro
65                  70                  75                  80

cac ctc cga gaa tat ctc cat tgg ttg gtg act gat atc cct gct aca      288
His Leu Arg Glu Tyr Leu His Trp Leu Val Thr Asp Ile Pro Ala Thr
                85                  90                  95

act gga aca acc ttt ggc aat gag att gtg tgt tac gaa aat cca agt      336
Thr Gly Thr Thr Phe Gly Asn Glu Ile Val Cys Tyr Glu Asn Pro Ser
            100                 105                 110

ccc act gca gga att cat cgt gtc gtg ttt ata ttg ttt cga cag ctt      384
Pro Thr Ala Gly Ile His Arg Val Val Phe Ile Leu Phe Arg Gln Leu
        115                 120                 125

ggc agg caa aca gtg tat gca cca ggg tgg cgc cag aac ttc aac act      432
Gly Arg Gln Thr Val Tyr Ala Pro Gly Trp Arg Gln Asn Phe Asn Thr

130                 135                 140

cgc gag ttt gct gag atc tac aat ctc ggc ctt ccc gtg gcc gca gtt       480
Arg Glu Phe Ala Glu Ile Tyr Asn Leu Gly Leu Pro Val Ala Ala Val
145                 150                 155                 160

ttc tac aat tgt cag agg gag agt ggc tgc gga gga aga aga ctt           525
Phe Tyr Asn Cys Gln Arg Glu Ser Gly Cys Gly Gly Arg Arg Leu
                165                 170                 175

<210>  2
<211>  175
<212>  PRT
<213>  Arabidopsis thaliana

<400>  2

Met Ser Ile Asn Ile Arg Asp Pro Leu Ile Val Ser Arg Val Val Gly
1               5                   10                  15

Asp Val Leu Asp Pro Phe Asn Arg Ser Ile Thr Leu Lys Val Thr Tyr
            20                  25                  30

Gly Gln Arg Glu Val Thr Asn Gly Leu Asp Leu Arg Pro Ser Gln Val
        35                  40                  45

Gln Asn Lys Pro Arg Val Glu Ile Gly Gly Glu Asp Leu Arg Asn Phe
    50                  55                  60

Tyr Thr Leu Val Met Val Asp Pro Asp Val Pro Ser Pro Ser Asn Pro
65                  70                  75                  80

His Leu Arg Glu Tyr Leu His Trp Leu Val Thr Asp Ile Pro Ala Thr
                85                  90                  95

Thr Gly Thr Thr Phe Gly Asn Glu Ile Val Cys Tyr Glu Asn Pro Ser
            100                 105                 110

Pro Thr Ala Gly Ile His Arg Val Val Phe Ile Leu Phe Arg Gln Leu
        115                 120                 125

Gly Arg Gln Thr Val Tyr Ala Pro Gly Trp Arg Gln Asn Phe Asn Thr
    130                 135                 140

Arg Glu Phe Ala Glu Ile Tyr Asn Leu Gly Leu Pro Val Ala Ala Val
145                 150                 155                 160

Phe Tyr Asn Cys Gln Arg Glu Ser Gly Cys Gly Gly Arg Arg Leu
                165                 170                 175

<210>  3

<211>  31
<212>  DNA
<213>  Artificial

<220>
<221>  Source
<223>  /note="Description of Artificial Sequence: Primer: FT-F"

<400>  3
cctctagaat gtctataaat ataagagacc c                                    31


<210>  4
<211>  27
<212>  DNA
<213>  Artificial

<220>
<221>  Source
<223>  /note="Description of Artificial Sequence: Primer: FT-R"

<400>  4
ctctgagcta aagtcttctt cctccgc                                         27

## Description

### TECHNICAL FIELD

The present invention relates to a transgenic plant capable of flowering in a low light environment, a method for producing the same, a method for inducing flowering of the plant, and so on. More particularly, the present invention relates to a transgenic plant capable of flowering in a low light environment (e.g., indoors) by introducing a FT gene, a method for producing the same, a method for inducing flowering of the plant, and so on.

### BACKGROUND ART

In recent years, it is well known that many genes are associated with control of flowering, mainly on Arabidopsis. Reportedly, a gene called FT gene derived from Arabidopsis plays an important role in the control of flowering and the flowering is promoted when the expression level of this FT protein increases, which is encoded by the gene (WO 99/53070, Trends in Plant Science (2006) 11: 550-558). It is known that the FT gene and the corresponding rice gene Hd3a are synthesized into a protein in a leaf and the protein moves to the shoot apex, where flower bud differentiation is then induced (Science (2007) 316: 1033-1036). When a transgenic plant wherein a FT gene is constitutively expressed is produced, flowering of the plant is promoted (Trends in Plant Science (2006) 11: 550-558: Non-Patent Document 1). For example, a FT gene-overexpressed chrysanthemum can flower even in tissue culture under long-day conditions that do not normally induce flowering of chrysanthemum (Horticultural Research (JAPAN) (2007) 6: supplementary volume 1, 214: Non-Patent Document 2).

Many plants contain genes corresponding to the FT gene. Orthologs of the FT gene are obtained from tomato and Populus trichocarpa and named SFT and PtFT, respectively. The same gene is also obtained from Citrus unshiu, etc. It is reported that when these genes are constitutively overexpressed in a plant, the flowering of Arabidopsis, rice, Poncitrus trifoliate, etc. were promoted (Plant Cell Physiol. (2002) 43: 1095-1105, Transgenic Research (2005) 14: 703-712: Non-Patent Document 3). In general, it takes several years from germination to flowering in trees such as fruit trees including citrus fruits but a transgenic orange into which the FT gene was introduced came into flowering in about six months to a year (Transgenic Research (2005) 14: 703-712: Non-Patent Document 4).

It is thus considered that the functions of FT gene and corresponding genes derived from other plant species would exerts its function of flowering induction in interspecies. Furthermore, when the FT gene and the corresponding genes from other plants (hereinafter collectively referred to as the FT gene) are used, it is expected to accelerate an alternation of generations and shorten the period required for cultivar improvement (JPA 000-139250: Patent Document 1). That is, when a plant in which the FT gene is introduced is cultivated under conditions of sufficient illumination and temperature, it is expected that anthesis would occur earlier than in non-transgenic plants. Alternatively, if a plant is maintained by tissue culture, it can be expected that flowering would be promoted and the plant would flower earlier than it does usually. The reason for the above is that in tissue culture sugar is generally added as the carbon and energy sources and the plant would grow even without conducting sufficient photosynthesis.

On the other hand, hours of sunshine are extremely important for the growth and flowering of plants. The intensity of outdoor illumination under clear sky is approximately 100,000 lux. When petunia or the like is cultivated in a greenhouse, the illumination intensity of about 10,000 lux is required. Turning to ordinary indoors (offices or homes), the illumination intensity is approximately 500 lux. African violet is only known as an ornamental plant which can flower under this condition.

Flowering potted plants (cyclamen, gerbera, etc.) are displayed indoors to appreciate flowers. Hence, there is a demand for appreciate flowers indoors. To meet this demand, plants which already flowered through induction in a greenhouse, etc. are usually sold for indoor ornamental purpose. If plants are exposed to sufficient light and appropriate day length, the plants will flower even indoors. Illumination intensity and a daily photoperiod vary depending upon plant species and cultivar. In general, when exposed to artificial illumination of about 1,000 lux such as by using a fluorescent lamp, etc., most plants can produce flowers. However, when ordinary flowering plants which require high light conditions for flowering are cultivated under insufficient light conditions, namely, in an ordinary indoor environment (e.g., an illumination intensity of approximately 200 to 600 lux, preferably approximately 500 lux and more preferably approximately 300 lux), these flower plants usually fail to produce flowers, or even if they flower, growth of the plants is inhibited and the plants exhibit yellowing or withering. This is because light is critical for photosynthesis and the photosynthesis is essential for the growth of plants.

In other words, notwithstanding that plants which can flower at low light intensity have been demanded, it still remains unclear if plants traditionally cultivated outsides would grow and flower under low light conditions like indoors, since light is an essential energy source for plant growth.
[Patent Document 1] JPA 2000-139250
[Non-Patent Document 1] Trends in Plant Science (2006) 11: 550-558
[Non-Patent Document 2] Horticultural Research (JAPAN) (2007) 6: supplementary volume 1,214
[Non-Patent Document 3] Plant Cell Physiol. (2002) 43: 1095-1105, Transgenic Research (2005) 14,703-712
[Non-Patent Document 4] Transgenic Research (2005) 14: 703-712

### DISCLOSURE OF INVENTION

### Problems To Be Solved

Under the foregoing circumstances, a transgenic plant capable of flowering in a low light environment and a method for producing the same have been demanded.

### Means To Solve The Problem

The inventors have conducted various experiments using FT genes being introduced into flowering plants and eventually accomplished the present invention. More specifically, the present invention relates to a method for producing a transgenic flowering plant capable of flowering in a low light environment, which comprises introducing a FT gene into a flowering plant, a flowering plant characterized by such a character, a kit comprising such a flowering plant as follows
(1) A method for producing a transgenic plant capable of flowering in a low light environment, which comprises introducing a FT gene into a flowering plant.
(2) The method according to (1) above, which comprises introducing the FT gene into a flowering plant to highly express the FT gene.
(3) The method according to (2) above, which comprises introducing the FT gene into a flowering plant to constitutively overexpress the FT gene.
(4) The method according to any one of (1) to (3) above, wherein the FT gene is derived from Arabidopsis.
(5) The method according to (4) above, wherein the FT gene is a variant that retains a flowering promoting function.
(6) The method according to any one of (1) to (5) above, wherein the constitutive expression of the FT gene is induced by a promoter derived from a plant virus.
(7) The method according to any one of (1) to (6) above, wherein the flowering plant is torenia of the family Scrophulariaceae, petunia of the family Solanaceae, Nierembergia of the family Solanaceae, verbena of the family Verbenaceae, rose of the family Rosaceae or carnation of the family Caryophyllaceae.
(8) The method according to any one of (1) to (7) above, wherein the low light environment is a cultivation environment under the lighting conditions wherein flowering is not caused in a plant of the same species into which a FT gene is not introduced.
(9) The method according to (8) above, wherein the low light environment has an illumination intensity of 50 to 1,000 lux.
(10) A transgenic plant produced by the method according to any one of (1) to (9) above.
(11) A method of inducing flowering of a plant in a low light environment, which comprises cultivating a transgenic flowering plant into which a FT gene is introduced in a lower light environment, as compared to that for cultivating a non-engineered flowering plant of the same species into which a FT gene is not introduced.
(12) The method according to (11) above, wherein the lower light environment is a cultivation environment under the lighting conditions wherein flowering is not caused in a plant of the same species into which a FT gene is not introduced.
(13) The method according to (12) above, wherein the lower light environment has an illumination intensity of 50 to 1,000 lux.
(14) The method according to any one of (11) to (13) above, wherein the conditions for cultivating the transgenic plant are conditions for tissue culture or soil culture.
(14a) The method according to any one of (1) through (14) above, wherein the plant does not cause any change to reduce its commercial value at least until flowering.
(14b) The method according to any one of (1) through (14) above, wherein the change to reduce its commercial value is a change in leaf color and/or petal color, yellowing or withering.
(14c) The method according to any one of (1) through (14) above, wherein the plant is capable of flowering in a shorter plant height than usual.
(15) A transgenic plant which is produced by the method recited in any one of (11) to (14) above, wherein the flowering plant is torenia of the family Scrophulariaceae, petunia of the family Solanaceae, Nierembergia of the family Solanaceae, verbena of the family Verbenaceae, rose of the family Rosaceae or carnation of the family Caryophyllaceae.
(16) A kit of a transgenic plant capable of flowering in a low light environment, comprising a transgenic flowering plant into which a FT gene is introduced.
(17) The kit according to (16) above, wherein the transgenic plant is a plant in the state of a seed or a seedling before flowering.

### Effect Of The Invention

According to the present invention, a plant usually incapable of flowering at low light intensity similar to an indoor environment can be induced to flower even in a low light environment by introducing the FT gene thereinto. Such plants capable of flowering at low light intensity are easily to culture indoors. Therefore, these plants can flower not only in the case that commercial growers cultivate them but in the case that general consumers do so indoors.

Further by constitutively expressing the FT gene, flowering plants can flower in a shorter period than they do, even in a low light environment.
Moreover, flowering plants can be cultivated to flower in a low light environment according to the method of the present invention. Thus, the method of the invention is advantageous in that energy consumption can be markedly reduced.

### SEQUENCE LISTING FREE TEXT

SEQ ID NO: 1: Arabidopsis thaliana FT gene sequence and protein sequence
SEQ ID NO: 2: Arabidopsis thaliana FT protein sequence
SEQ ID NO: 3: Primer
SEQ ID NO: 4: Primer

### BEST MODES FOR CARRYING OUT THE INVENTION

Hereinafter, detailed description is given on the FT gene used in the present invention, a polypeptide encoded by the same, a vector for expressing the FT gene, a method for producing the transformant, a method for tissue culturing the transformant to a plant body, cultivation, and so on.

### 1. Transgenic plant and method for producing the same according to the invention

### 1.1 FT gene used in the invention

First, the present invention provides a method for producing a transgenic flowering plant capable of flowering in a low light environment, which comprises introducing the FT gene into the flowering plant. According to this method, the FT gene described above is preferably introduced into the flowering plant so as to highly express the gene. More preferably, the FT gene is introduced into the flowering plant so as to constitutively overexpress the gene.

As used herein, the "FT gene" refers to, but not limited to, the gene (SEQ ID NO: 1, Genbank Accession No. AB027504) encoding the FT protein (SEQ ID NO: 2) derived from Arabidopsis (Arabidopsis thaliana) and may be genes for orthologous proteins belonging to the same family as FT which show the flowering promoting activity of the FT protein. Proteins belonging to the same family as FT are found in many plants and highly conserved in the whole family. Proteins in the same family as FT include FT protein, TSF protein and MFT protein of Arabidopsis thaliana, CiFT protein of Citrus unshiu, and Hd3a protein, RFT1 protein, AL662946 protein, AP003079 protein and AP002882 protein of rice. Herein, the numbers in AL662946 protein, AP003079 protein and AP002882 protein are the registration numbers for ESTs of rice origin.

As used herein, the term "polynucleotide" is used interchangeably with "gene," "nucleic acid" or "nucleic acid molecule" and refers to a polymer of nucleotides. As used herein, the term "nucleotide sequence" is used interchangeably with "nucleic acid sequence" or "nucleotide sequence" and represents a sequence of deoxyribonucleotides (abbreviated as A, G, C and T). The "polynucleotide comprising the nucleotide sequence of SEQ ID NO: 1 or a fragment thereof" is intended to mean a polynucleotide comprising the sequence represented by deoxyribonucleotides A, G, C and/or T of SEQ ID NO: 1, or a fragment thereof.

The polynucleotide according to the present invention may be in the form of RNA (for example, mRNA) or DNA (for example, cDNA or genomic DNA). The DNA may be double stranded or single stranded. The single strand DNA or RNA may be a coding strand (also known as a sense strand) or a non-coding strand (also known as an anti-sense strand).

The source for acquiring the polynucleotide according to the present invention is not particularly limited and preferably a biological material containing the FT gene or an ortholog thereof. As used herein, the term biological material is intended to mean a biological sample (a tissue sample or cell sample obtained from a living organism). In EXAMPLES described below, petunia, torenia and Nierembergia are used as the organism but not limited thereto.

In one embodiment, the polynucleotide according to the present invention may be a mutant in which one or more nucleotides are deleted, inserted, substituted or added in the nucleotide sequence of a polynucleotide encoding the polypeptide having a FT protein activity. The mutant may have a mutation in the coding region or non-coding region, or both of these regions. The mutation in the coding region may be deletion, insertion, substitution and/or addition of amino acids, which may be conservative or non-conservative. As used herein, "the FT gene" includes a gene encoding the amino acid sequence of SEQ ID NO: 2 and a gene encoding an amino acid sequence in which one or more (e.g., 1-40, 1-20, 1-15, 1-10, 1-9, 1-8, 1-7, 1-6, 1-5, 1-4, 1-3, 1-2, 1, etc.) or one or several amino acids are deleted, substituted or added in said amino acid sequence.

It is well known in the art that some amino acids in the amino acid sequence of the polypeptide may easily be modified without causing any significant effect on the structure or function of the polypeptide. It is also well known that not only in artificially modified proteins but also in naturally occurring ones, there are mutants in which the structure or function of the protein is not significantly altered.

A person of ordinary skill in the art can easily modify one or more amino acids in the amino acid sequence of the polypeptide by applying well-known techniques. For example, a given nucleotide in the polynucleotide encoding the polypeptide can be altered by known point mutagenesis methods. Further by designing a primer corresponding to a given site of the polynucleotide encoding the polypeptide, deletion mutants or addition mutants can be prepared. Furthermore, whether or not the prepared variants have the desired activity can be easily assayed by using the methods described herein.

Preferred variants contain conservative or non-conservative amino acid substitutions, deletions or additions, which are preferably silent substitutions, additions and deletions, and particularly preferably conservative substitutions. These variants do not alter the activity of the polypeptide according to the present invention.

The representative examples of conservative substitution include replacement of another amino acid for one amino acid among aliphatic amino acids Ala, Val, Leu and Ile; exchange between the hydroxyl residues Ser and Thr, exchange between the acidic residues Asp and Glu, replacement between the amide residues Asn and Gln, exchange between the basic residues Lys and Arg, and replacement between the aromatic residues Phe and Tyr.

As described above in detail, a further guidance on which amino acid alteration could be phenotypically silent (namely, which amino acid alteration could hardly exert significantly harmful effects on the function) can be found in Bowie, J. U., et al., "Deciphering the Message in Protein Sequences: Tolerance to Amino Acid Substitutions," Science 247: 1306-1310 (1990), which is incorporated herein by reference.

By using the above-described polynucleotide according to the present invention, the polypeptide carrying the function of the FT protein can be synthesized in transformants or cells.

### 1.2 Expression vector

The present invention provides a method which comprises introducing the FT gene into a flowering plant to highly express the FT gene. The invention further provides a method which comprises introducing the FT gene into a flowering plant to constitutively overexpress the FT gene.

As used herein, the term "high expression" refers to a state where the gene expressively incorporated functions more vigorously per cell, on the grounds that the gene is induced for expression more strongly or accumulates in a cell for a longer period of time than in the original conditions. However, the grounds are not limited to these cases. The term "constitutive overexpression" refers to overexpression at an almost constant level wherein the gene expression is independent of external stimuli or growth conditions, etc. The gene "expressively incorporated" is intended to mean a gene which is incorporated into a vector in a manner that the gene can be expressed in a cell into which the gene is introduced. The gene "expressively incorporated (into a vector)" can be expressed by placing an expression regulating factor such as a suitable promoter for regulating expression of the gene at a position capable of regulating expression of the gene in the vector (e.g., an upstream region of the gene).

As used herein, "the FT gene construct capable of expressing in a plant" refers to a construct in which the FT gene is inserted in such a form capable of introducing the same into a plant by known gene transfer methods (e.g., a plasmid vector) and is intended to mean a construct that is capable of expressing the FT protein encoded by the FT gene. The FT gene construct in such a form that the FT gene can be introduced into a plant includes, for example, a recombinant expression vector into which cDNA of the FT protein is inserted, and the like. Methods for preparing the recombinant expression vector include, but not limited to, methods using plasmid, phage, cosmid, etc. known to those skilled in the art.

Specific types of vectors are not particularly limited but vectors which can be expressed in host cells may be appropriately employed. Depending on the type of host cells, a promoter sequence is appropriately chosen to ensure expression of the FT protein and a vector prepared by introducing the promoter sequence and the FT gene into a certain type of plasmid may be used as an expression vector.

Preferably, the vector contains at least one selection marker. Examples of selections marker for plant cell incubation include kanamycin resistance gene, hygromycin resistance gene and Basta resistance gene, and those for incubation of E. coli and other bacteria include tetracycline resistance gene, ampicillin resistance gene, gentamicin resistance gene, kanamycin resistance gene, bleomycin resistance gene, chloramphenicol resistance gene, and the like. Using the selection marker, a plant body which grows in a medium containing the antibiotic described above is selected, whereby the transgenic plant body and bacteria can be easily selected.

An expression promoter of the plasmid may be one that induces a constitutive expression or may also be an externally-regulatable induction type promoter. Examples of the promoter capable of inducing constitutive expression which can be preferably used in the present invention include a cauliflower mosaic virus 35S promoter (CaMV35S), an actin promoter, a promoter of nopaline synthase, a tobacco PR1a gene promoter, a tomato ribulose-1,5-bisphosphate carboxylase oxidase small subunit promoter, etc. Among these, cauliflower mosaic virus 35S promoter and actin promoter may be used more preferably. By using any of the promoters described above, a given gene can be strongly expressed in the obtained recombinant expression vector when the gene is introduced into a plant cell. Examples of the externally regulatable promoter include, but not limited to, a metallothionein promoter, a heat shock promoter, etc.

According to the present invention, the induction type promoter, when specifically binding to a DNA-binding domain, is activated by the effect of a transcriptional activation domain to transcribe a structural gene under the control thereof. In the present invention, the induction type promoter is appropriately selected depending on a sequence used as a DNA sequence coding for the DNA-binding domain. For example, when a DNA sequence encoding the DNA-binding domain (amino acids 1 to 82) of mouse AhR is used as the DNA sequence encoding the DNA-binding domain, 6 x XRE sequence (a sequence in which six mouse XRE sequences are linked in tandem) derived from E. coli is preferably used. When the DNA sequence encoding the DNA-binding domain of E. coli repressor LexA is used as the DNA sequence encoding the DNA-binding domain, 8 x LexA-46-P sequence (LexA-binding sequence repeated 8 times in series in an operator region (46 nucleotides upstream from the transcription initiation site) of cauliflower mosaic virus 35S promoter) is preferably used.

In a preferred embodiment, the vector of the present invention may further comprise one or more desirable genes. Examples of the genes include drug resistance genes (e.g., NPTII (neomycin phosphotransferase II)) and the like. These genes are placed preferably under the control of an appropriate promoter (e.g., a nopaline synthase promoter or a Mac promoter).

In general, the vector of the present invention contains an expression regulatory domain such as an appropriate promoter and a terminator as well as an origin of replication depending on type of host into which the vector is to be introduced. For example, in order to express a gene in a plant cell, a DNA molecule (expression cassette ), is prepared so as to have (1) a promoter which can function in the plant cell, (2) the gene and (3) a terminator which can function in the plant cell in a functional form, and introduced into the plant cell. Such a DNA molecule may contain a DNA sequence to further enhance transcription, e.g., an enhancer sequence, in addition to the promoter. The promoter used is not particularly limited as long as it is capable of functioning in plant cells and includes, for example, 35S (Schell, J. S., 1987, Science, 237: 1176-1183), Nos (Schell, J. S., 1987, Science, 237: 1176-1183), rbcS (Benefy, P. N. and N-H, Chua, 1989, Science, 244: 174-181), PR1a (Ohshima, M. et al., 1990, Plant Cell, 2: 95-106), ADH (Benefy, P. N. and N-H, Chua, 1989, Science, 244: 174-181), patatin (Benefy, P. N. and N-H, Chua, 1989, Science, 244: 174-181), Cab (Benefy, P. N. and N-H. Chua, 1989, Science, 244: 174-181) and PAL (Lian, X. et al., 1989, Proc. Natl. Acad. Sci. USA, 86: 9284-9288), etc.

The terminator is not particularly limited as far as it functions as a transcription termination site, and may be one known in the art. Specific examples of the terminator which can be preferably employed include nopaline synthase gene transcription termination region (Nos terminator), cauliflower mosaic virus 35S transcription termination region (CaMV35S terminator), and the like. Among these, Nos terminator can be used more preferably.

The nucleotide sequence to enhance translation efficiency includes, for example, an omega sequence from tobacco mosaic virus. By placing this omega sequence in the untranslated region (5' UTR) of the promoter, translation efficiency of the subject gene can be increased. As described above, the transformation vector may contain various DNA segments according to the purpose.

The method of constructing the recombinant expression vector described above is not particularly limited. The promoter described above, a gene encoding a transcription factor, a transcription repressor converting polynucleotide and, if necessary, other DNA segments are introduced into a suitably selected carrier vector in a predetermined order. For example, the gene encoding the transcription factor is ligated to the transcription repressor converting polynucleotide to construct a chimeric gene. The chimeric gene is then ligated to a promoter (and a terminator, etc., if necessary) to construct an expression cassette and the cassette is inserted into a vector.

In constructing the chimeric gene and the expression cassette, the order of DNA segments can be regulated, for example, by providing, at the cleavage sites of each DNA segment, protruding ends that are complementary each other, and then reacting them with a ligase. In the case that the expression cassette contains a terminator, it may contain a promoter, the chimeric gene described above and the terminator in the order mentioned from the upstream side. The types of reagents used to construct the recombinant expression vector, such as restriction enzymes and ligase, etc., are not particularly limited and may be suitably selected from commercially available products.

### 1.3 Preparation of transgenic plant

According to the present invention, the transgenic flowering plant that is transformed as described above is provided.

As used herein, the term "flowering plant" collectively refers to a plant which produces flowers. The flowering plant is preferably intended to mean an ornamental horticultural plant, and more preferably, an ornamental flowering plant. The flowering plant may be an angiosperm or a gymnosperm.

As used herein, the term "transgenic plant" is interchangeably used with "a transformed plant."

Known methods for gene transfer includes but not limited to the Agrobacterium-mediated method, particle bombardment method, virus vector method, electroporation method, polyethylene glycol method, microinjection method, liposome method, and the like. A person of ordinary skill in the art may choose a preferred gene transfer method depending on the type of the plant into which the gene is introduced.

Construction of the vector described above can be performed in a conventional manner using known restriction enzymes, etc. In the case using, e.g., Agrobacterium, a binary vector such as pBI121 may be used, whereas an Escherichia coli vector such as pUC19 may be used in the case using a particle gun. Furthermore, a plant transformed with a target gene may be obtained by selecting plant cells transformed with the vector using a marker gene such as an antibiotic resistance gene and redifferentiating the selected cells using an appropriate plant hormone or other conditions.

The vector can be introduced into a plant cell by various methods known to those skilled in the art. Examples of the methods which can be used include indirect introduction methods using Agrobacterium tumefaciens or Agrobacterium rhizogenes (Heiei, Y. et al., Plant J., 6: 271-282, 1994; and Takaiwa, F. et al., Plant Sci. 111: 39-49, 1995), and direct introduction methods represented by the electroporation method (Tada, Y et al., Theor. Appl. Genet, 80: 475, 1990), the polyethylene glycol method (Datta, S. K. et al., Plant Mol. Biol., 20: 619-629, 1992), and the particle gun method (Christou, P. et al., Plant J., 2: 275-281, 1992, Fromm, M. E., Bio/Technology, 8: 833-839, 1990), etc. The plant cell used for the gene transfer according to the present invention is not particularly limited so long as the cell can be regenerated into a plant. Examples of the plant cell include suspension culture cells and cells capable of forming calluses, protoplasts, leaf slices, etc.

The transformed plant cell can be regenerated to produce a plant. Reference is made to literatures including Ueyama, et al., Plant Biotechnology, 23: 19-24, 2006; Tamura, et al., Plant Cell Reports, 21: 459-466, 2002; Aida, et al., Biotechnology in Agriculture and Forestry, 48: 294- Transgenic Crops III (ed. by Y.P.S. Bajaj) Springer-Verlag Berlin Heidelberg 2001; the method of Horsh, et al. (Science. 1985, 227: 1229-1231); and the method of Hazama, et al. (Biotechnology in Agriculture and Forestry, 48: Transgenic Crops III, Springer-Verlag Berlin Heidelberg 2001), etc.

The present invention provides the transgenic plant produced by the methods described above. Preferred plant species which are transformed by the methods of the present invention described above include flowering plant species. Plant species from which transformants can be acquired are more preferred. Examples of such plant species are petunia, torenia, verbena, tobacco, rose, chrysanthemum, carnation, snapdragon, cyclamen, orchid, lisianthus, freesia, gerbera, gladiolus, soaproot, kalanchoe, lily, pelargonium, geranium, tulip, marigold, Nierembergia, morning glory, etc. (see, e.g., Chandler, et al., In Vitro Cellular and Developmental Biology Plant, 41: 591-601, 2005; and Tanaka et al., Genetic engineering in floriculture, Plant Cell, Tissue and Organ Culture, 80: 1-24, 2005).

The transgenic plant according to the present invention is a transgenic plant which can flower in such a cultivation environment that an illuminance level does not exceed 1,000 lux (preferably a low light indoor condition of 100 lux or less) by introducing the FT gene thereinto.

Both plants modified by introducing the vector of the invention thereinto and plants obtained from the productive materials of said plants (e.g., seed, posterity, cut flower, tuberous root, tuber, fruit, cutting, etc.) are also included in the technical scope of the invention.

In the method for producing a plant according to the present invention, the gene described above is introduced into the plant, thereby the posterity in which the level is simply reduced can be acquired by sexual reproduction or asexual reproduction from said plant. Furthermore, the plant cells or productive materials such as seeds, fruits, roots, tubers, cuttings and tuberous roots can be obtained from the plant and its posterity to mass-produce said plant. Therefore, the method for producing the plant according to the present invention may contain a productive step (mass-production) to reproduce the plant after selection.

In the present invention, the plant includes at least one of grown plants, plant cells, plant tissues, calluses and seeds. In other words, any material that can finally grow to plant individuals is regarded as the plant. The plant cells described above include plant cells of various forms. For example, suspension culture cells, protoplasts, leaf slices and the like are included in these plant cells. By growing and differentiating these plant cells, a plant can be obtained. The plant can be regenerated from plant cells by known methods, depending on the type of the plant cells. Accordingly, the method for producing the plant according to the present invention may contain a regeneration step to regenerate the plant from plant cells.

### 2. Method for inducing flowering of the transgenic plant of the present invention

Next, the present invention further relates to a method for inducing flowering of a plant in a low light environment, which comprises cultivating a transgenic flowering plant into which the FT gene is introduced in a lower light environment, as compared to that for cultivating a non-engineered flowering plant of the same species into which the FT gene is not introduced.

Specifically, the "low light environment" as used herein means a cultivation environment under the lighting conditions that a plant of the same species into which the FT gene is not introduced does not flower. Such environment includes, for example, one with an illumination level lower by 10 to 70%, an illumination level lower by 10 to 50%, an illumination level lower by 10 to 40% and an illumination level lower by 10 to 30%, than that for a non-engineered flowering plant of the same species into which the FT gene is not introduced, in terms of the total illumination level until flowering. Herein, the "illumination level lower by 10 % than that for a non-engineered flowering plant of the same species into which the FT gene is not introduced, in terms of the total illumination level until flowering" is intended to mean that the exposure to an average illuminance of 10,000 lux (10-hour light period) for 10 days (10,000 x 10 x 10 = 1,000,000 lux hour) is required for the non-engineered flowering plant to flower, whereas the exposure to the illumination level of 9,000 lux (10-hour light period) for 10 days (9,000 x 10 x 10 = 900,000 lux hour) is sufficient for the transgenic plant.

More specifically, in a preferred embodiment of the invention, ornamental flowering plants which can usually flower outdoors only can be induced to flower even under dark illumination conditions like that of indoors.

Such a low light environment varies depending on the type of the plant, type of the FT gene to be introduced, the type of the promoter or vector used, cultivation conditions including temperature but generally refers to the following conditions:

Illumination conditions for the tissue culture conditions or in the ordinary soil culture conditions include, for example, a cultivation environment at a temperature of 10 to 35°C under a light period of 50 to 1,000 lux for 10 to 18 hours, more preferably under a light period of 100 to 500 lux for 10 to 18 hours

Preferred cultivation and illumination conditions vary depending on the type of the flowering plant. Torenia, petunia and Nierembergia are long-day plants, and similar conditions are applicable to these plants. The conditions are as follows:

For example, a plant is placed in a vessel charged with a culture medium including a Murashige and Skoog basal medium (Duchefa Biochemie) supplemented with 3 g/l Gellan Gum (Kanto Chemical), 30 g/l sucrose and 4.4 g/l vitamins, and tissue culture can be performed by cultivating at about 22°C under the illuminance of about 1,000 lux (e.g., 100 to 1,200 lux, preferably 200 to 1,000 lux) (using a fluorescent lamp, 16-hour light period and 8-hour dark period).

Furthermore, a plant can be planted in ordinary horticultural soil and cultivated outdoors. For the outdoor cultivation, it is desirable that a minimum temperature is 10 to 15°C or higher, a maximum temperature is 35°C or lower and duration of sunshine for 10 to 12 hours or more. For the indoor cultivation, artificial cultivation soils such as Ecopuff, Hydroball, Supersol, etc. tend to be more popular among consumers than ordinary soils, but horticultural solids may also be used. In the present invention, the term "soil culture" is intended to mean cultivation using natural soil or artificial soil. The soil culture includes a variety of cultivation environment such as open cultivation, greenhouse cultivation, plastic greenhouse cultivation, and the like.

The cultivation conditions used in the present invention are not particularly limited to the conditions described above.

### 3. Kit of the transgenic plant capable of flowering in a low light environment

The present invention further provides a kit of the transgenic plant capable of flowering in a low light environment. This kit of the transgenic plant comprises the transgenic plant in the state of a seed or a seedling before flowering.

The plant which is suitable for the plant kit according to the present invention is preferably an ornamental flowering plant. Any flowering plant may be available for the flowering plant which can be used in the present invention, so long as transformation techniques are applicable thereto, and a person of ordinary skill in the art may readily choose an ornamental flowering plant to which transformation techniques are easily applicable. Preferably, the transgenic flowering plant of the present invention is a plant that maintains its stable character for breeding which is transmissible to its progenies.

The kit according to the present invention is preferably supplied with operating instructions which describes appropriate conditions for cultivating the transgenic flowering plant of the present invention, and fertilizers to be used for cultivation. Particularly preferred illumination condition is an illumination sufficient for interior lighting.

By using the plant kit according to the present invention, general consumers can easily cultivate the plant in houses and offices, allowing the plant to flower in a low light environment.

All publications mentioned herein are incorporated herein by reference in their entirety. The invention is described with reference to specific preferred embodiments but various modifications and alterations of the invention will become apparent without departing from the scope and spirit of the invention. These modifications and alterations are intended to be within the scope of the appended claims.

Hereinafter, the present invention will be described in more detail by referring to EXAMPLES but is not deemed to be limited to thereto.

### EXAMPLES

In the following EXAMPLES, molecular biological procedures were carried out, in accordance with WO 96/25500 and Molecular Cloning (Sambrook et al. (1989) Cold Spring Harbour Laboratory Press), unless otherwise indicated.

### EXAMPLE 1: Construction of Expression Vector

Based on the FT gene described in WO 99/53070, the following two primers were synthesized:
FT-F 5'-CCTCTAGAATGTCTATAAATATAAGAGACCC-3' (SEQ ID NO: 3),
FT-R 5'-CTCTGAGCTAAAGTCTTCTTCCTCCGC- 3' (SEQ ID NO: 4)
Arabidopsis was cultivated under long day conditions to harvest mRNA. Using this mRNA as a template, FT cDNA was synthesized by RT-PCR using the two primers described above. RT-PCR was performed, using KOD-Taq (Toyobo) under the conditions recommended by the manufacturer. The synthesized cDNA was digested with XbaI. On the other hand, p35S-GFP Plant Vector (CLONTECH) was digested with BamHI and SacI and blunted using a DNA Blunting Kit (Takara Bio), followed by self ligation. After the resulting plasmid was digested with XbaI, the above-described FT cDNA after digestion with XbaI was inserted therein. A plasmid in which the initiation codon of FT cDNA was inserted adjacent to cauliflower mosaic virus 35S promoter of p35S-GFP was selected. This plasmid was digested with HindIII and EcoRI. Among the resulting DNA fragments, the DNA fragment carrying cauliflower mosaic virus 35S promoter, the FT gene and nopaline synthase terminator were inserted into pBinPlus (Transgenic Research, 1995, 4, 288-290) digested with HindIII and EcoRI. The plasmid obtained was named pSPB3137. By using pSPB3137, it is expected that FTcDNA is regulated by cauliflower mosaic virus 35S promoter and constitutively expressed in a plant.

### EXAMPLE 2: Production and Evaluation of Transgenic Petunia

Transformation was performed according to the method of Horsh et al. (Science, 1985, 227:1229-1231). In brief, a binary vector carrying the T-RNA segment constructed in EXAMPLE 1 was introduced into Petunia hybrida cultivar Surfinia Purple Mini (Suntory Flowers, Ltd.) by the Agrobacterium method using a leaf disc. The transgenic cells were selected with kanamycin and regenerated to obtain transgenic petunias. Some of the transgenic petunias produced flower under in vitro cultivation at an illumination of 5,000 lux (16-hour light period and 8-hour dark period) at a temperature of 23°C, whereas petunias in which the gene was not introduced or transgenic petunias in which other genes were introduced did not produce flower under the same conditions.

The transgenic petunias in which the FT gene was introduced were planted in a 10 cm-diameter plastic pot charged with a mixture of BM1 (peat moss) : akadama soil : perlite = 2 : 2 : 1 and cultivated in a contained greenhouse. RNA was extracted from the leave of each transgenic petunia. The presence or absence of the FT transcript was examined by RT-PCR to select the line in which the FT gene was transcribed. Primers used to detect FT mRNA were the same as in the EXAMPLE. Four rooted cuttings were prepared from each line where the FT mRNA was detected, and were planted in a mixture of BM1 (peat moss) : akadama soil : perlite = 2 : 2 : 1. Cultivation was continued in the contained greenhouse. The transgenic petunia in which FT was introduced flowered on days 25 to 48, whereas the non-transgenic petunia in which FT was not introduced flowered on days 64 to 79.

The foregoing results reveal that flowering of petunia can be accelerated by overexpression of the FT gene, both under tissue culture conditions and ordinary soil culture.

### EXAMPLE 3: Production and Evaluation of Transgenic Torenia

Transformation of torenia was performed according to the method of Hazama, et al. (Biotechnology in Agriculture and Forestry, 48: Transgenic Crops III, Springer-Verlag Berlin Heidelberg 2001). In brief, a binary vector carrying the T-DNA segment constructed in EXAMPLE 1 was introduced into Torenia hybrida cultivar Summer Wave Blue (Suntory Flowers, Ltd.). The transgenic cells were selected with kanamycin and regenerated to obtain transgenic torenias. Some of the transgenic torenias flowered under in vitro cultivation at an illumination of 5,000 lux (16-hour light period and 8-hour dark period) at a temperature of 23°C, whereas torenias in which the gene was not introduced or transgenic torenias in which other genes were introduced did not flower under the same conditions.

RNA was extracted from the leaf of 40 lines of the torenia in which the FT gene was introduced. The presence or absence of the FT transcript was examined by RT-PCR to select the line in which the FT gene was transcribed.

The transgenic torenias were planted in a 10 cm-diameter plastic pot charged with a mixture of BM1 : akadama soil : perlite = 2 : 2 : 1 and cultivated in a contained greenhouse. The transgenic torenias in which FT was introduced flowered on days 33 to 48, whereas the non-transgenic torenias in which FT was not introduced flowered on days 60 to 63. Tendencies with shortened internodes and compact plant types were observed for the transgenic petunia.

The foregoing results reveal that flowering of torenia can be accelerated both under tissue culture conditions and ordinary soil culture, by constitutively expressing the FT gene and overexpressing the FT protein.

### EXAMPLE 4: Flowering of Transgenic Torenia at Low Light Intensity

The cuttings from the transgenic and host torenias were planted in Paffcal (Suntory Ltd., described in WO 2004/112461). These cuttings were placed in a foam carton with pores and floated on water containing a diluted liquid fertilizer (e.g., a 1,000-fold dilution of Vigor Life, Suntory Flowers, Ltd.). These torenias were cultivated for 3 months under conditions at an illumination of 3 00 lux (16-hour light period and 8-hour dark period) and a temperature of 23°C. Under the conditions, the host torenias were observed to grow to develop new leaves but no flower bud was formed and the host torenias did not result in flowering. Turning to the transgenic torenias wherein the FT gene was introduced, flower buds were formed on days 25 to 60 and the transgenic torenias flowered on days 52 to 70. In this case, any abnormality was not observed in the color or morphology of leaves and flowers. As described in EXAMPLE 3, however, the internodes were shortened when compared with the host and the plant type was compact, and the appearance of flowering was very noble and pretty.

Plants can flower under the in vitro conditions that exposure to light is sufficient and sucrose is supplied as the carbon source. However, there are few plants capable of flowering in a room where not much light is available and the carbon source is also limited. Even if a signal (e.g., overexpression of the FT gene) is added to promote flowering, it is considered that a certain level of light to drive sufficient photosynthesis is normally required for flowering of plants. Therefore, it is unpredictable if plants can flower indoors only by transfer of the FT gene. However, surprisingly, the transgenic torenia flowered under low light conditions in EXAMPLE 4 and had a commercially attractive morphology.

### EXAMPLE 5: Production and Evaluation of Transgenic Nierembergia

A binary vector carrying the T-DNA segment constructed in EXAMPLE 1 was introduced into Nierembergia sp. cultivar Fairy Bells Patio Blue (Suntory Flowers, Ltd.) by the method of Ueyama, et al. (Plant Biotechnology, 23:19-24,2006) to obtain a transgenic Nierembergia. The host needed approximately 2 months from conditioning to flowering, whereas many of the transgenic Nierembergias exhibited earlier flowering by approximately 7 to 10 days than the host. The results above indicate that flowering of Nierembergia can be promoted by constitutive expression of the FT gene to overexpression the FT protein.

### EXAMPLE 6: Evaluation of FT-Introduced Transgenic Torenia by Hydroculture

Hydroculture (a method which involves storing water in a planter having no hole at the bottom and cultivating a plant therein) is widely used for indoor plant cultivation. For plant cultivation, hydroballs are used in place of soils. Hydroballs used in this EXAMPLE were Neocoal (registered trademark of Toyo Denka Kogyo Co., Ltd.). Rooted torenia cuttings were transplanted to approximately 20 ml of Neocoal charged in a 30 ml tube and cultivation was started. A 2000-fold dilution of Vigor Life V (registered trademark of Suntory Flowers, Ltd.) was supplied each time Neocoal dried out. Under the illumination conditions described in EXAMPLE 4, the transgenic torenias in which the FT gene was introduced produced flower buds in a month to 6 weeks, whereas the formation of flower buds was not observed in the host torenias. After these plants grew up, the stems were once cut back to the length of about 5cm. Then, the cultivation was continued. The transgenic torenias flowered in about a month and kept flowering while continuing growth. On the other hand, the host torenias did not flower at all.

### EXAMPLE 7: FT Gene Transfer into Rose

Using the Agrobacterium in which pSPB3137 was introduced, the FT gene was introduced into rose cultivar Lavande. Many methods for transformation of roses have already been reported (e.g., Firoozababy, et al., Bio/Technology 12:883-888 (1994); US 5480789; US 5792927; EP 536 327 A1; and US 20010007157 A1). The transformation can be performed according to these methods.

In brief, rose calluses induced from the leaf of sterile seedlings were immersed for 5 minutes in a bacteria solution of the Agrobacterium tumefaciens Ag10 strain (Lazo, et al., Bio/Technology, 9: 963-967, 1991) into which pSPB3137 was introduced. After an excess of the bacteria solution was wiped off with a sterile filter paper, the calluses were transplanted to a subculture medium and co-cultivated for 2 days in the dark.

Thereafter, the calluses were washed with MS liquid medium supplemented with 400 mg/l carbenicillin, and then transplanted to a selection/sterilization medium supplemented with 50 mg/l kanamycin and 200 mg/l carbenicillin. Transplantation and cultivation of the part which was not affected by growth inhibition but proliferated normally were repeated on the selection medium to select kanamycin-resistant calluses.

### EXAMPLE 8: FT Gene Transfer into Carnation

The plasmid pSPB3137 described above was digested with AscI and PacI. The resulting fragment carrying the FT gene was recovered. This fragment was ligated to the binary vector pCGP1988 (described in WO 2004-020637) digested with AscI and PacI. The plasmid obtained was named pSPB3432. This binary vector contains SurB gene as a marker for plant transformation.

Using the Agrobacterium tumefaciens Ag10 strain in which pSPB3432 was introduced, the FT gene was introduced into carnation cultivar Vega. The procedures for gene transfer to carnation are described in PCT/AU94/00265, and Bio/Technology, 9: 864-868 (1991), etc.

### INDUSTRIAL APPLICABILITY

According to the present invention, plants capable of flowering indoors were developed by introducing the FT gene to promote flowering. These plants are useful for business concerning indoor flowering plants, e.g., business concerning indoor cultivation. The present invention is also useful for business of indoor ornamental plants because it provide with a means to develop more attractive indoor ornamental plants to attract consumers.

## Claims

1. A method for producing a transgenic plant capable of flowering in a low light environment, which comprises introducing a FT gene into a flowering plant.

2. The method according to claim 1, which comprises the step of introducing the FT gene into a flowering plant to highly express the FT gene.

3. The method according to claim 2, which comprises the step of introducing the FT gene into a flowering plant to constitutively overexpress the FT gene.

4. The method according to any one of claims 1 to 3, wherein the FT gene is derived from Arabidopsis.

5. The method according to claim 4, wherein the FT gene is a variant retaining a flowering promoting function.

6. The method according to any one of claims 1 to 5, wherein the constitutive expression of the FT gene is induced by a promoter derived from a plant virus.

7. The method according to any one of claims 1 to 6, wherein the flowering plant is torenia of the Scrophulariaceae, petunia of the Solanaceae, Nierembergia of the Solanaceae, verbena of the Verbenaceae, rose of the Rosaceae or carnation of the Caryophyllaceae.

8. The method according to any one of claims 1 to 7, wherein the low light environment is a cultivation environment under the lighting conditions wherein flowering is not caused in a plant of the same species into which a FT gene is not introduced.

9. The method according to claim 8, wherein the low light environment has an illumination intensity of 50 to 1,000 lux.

10. A transgenic plant produced by the method according to any one of claims 1 to 9.

11. A method of inducing flowering of a plant in a low light environment, which comprises the step of cultivating a transgenic flowering plant into which a FT gene is introduced in a lower light environment, as compared to that for cultivating a non-engineered flowering plant of the same species into which a FT gene is not introduced.

12. The method according to claim 11, wherein the lower light environment is a cultivation environment under the lighting conditions wherein flowering is not caused in a plant of the same species into which a FT gene is not introduced.

13. The method according to claim 12, wherein the lower light environment has an illumination intensity of 50 to 1,000 lux.

14. The method according to any one of claims 11 to 13, wherein the conditions for cultivating the transgenic plant are conditions for tissue culture or soil culture.

15. A transgenic plant which is induced to flower by the method recited in any one of claims 11 to 14, wherein the flowering plant is torenia of the Scrophulariaceae, petunia of the Solanaceae, Nierembergia of the Solanaceae, verbena of the Verbenaceae, rose of the Rosaceae or carnation of the Caryophyllaceae.

16. A kit of a transgenic plant capable of flowering in a low light environment, comprising a transgenic flowering plant into which a FT gene is introduced.

17. The kit according to claim 16, wherein the transgenic plant is a plant in the state of a seed or a seedling before flowering.
